# EUROPEAN PATENT SPECIFICATION

(11) **EP 2 168 477 B1**
(45) Date of publication and mention of the grant of the patent: **24.05.2017**
(21) Application number: 09170741.4
(22) Date of filing: 18.09.2009
(51) Int. Cl.: A61B 5/0408, A61B 5/0424

(54) **ECG system and method for proper electrode placement**
EKG-System sowie Verfahren zur korrekten Elektrodenanbringung
Système ECG et procédé de placement correct d'électrodes

(30) Priority: 26.09.2008 DE 102008049287
(43) Date of publication of application: 31.03.2010
(73) Proprietor: eResearch Technology GmbH, 97230 Estenfeld (DE)
(72) Inventor: Reinstädtler, Jürgen, 97082 Würzburg (DE)
(74) Representative: Hellmich, Wolfgang

(56) References cited:
- EP-A1- 0 450 350
- WO-A1-99/40844
- WO-A2-2006/097085
- WO-A2-2008/015667
- US-A1- 2003 153 840

## Description

The invention relates to the field of ECG systems according to the preamble of claim 1 and to a method according to the preamble of claim 5. In medical science various measuring techniques are known to determine parameters of a patient's body. For the present application, above all the picking off of electric potentials from the upper part of the human body in order to obtain electrocardiograms (ECGs) is relevant.

In order to obtain an electrocardiogram (ECG) the bioelectric potentials or potential differences which occur during the depolarization and repolarization of the heart are recorded. The measurement of the potentials may be performed bipolar or unipolar by means of electrodes on the body surface.

Apparatus for acquiring ECGs fall under the generic term diagnostic apparatus. However, for example, a defibrillator likewise measures an ECG. Therefore, for the purposes of the present application, therapeutic apparatus which measure bioelectric potentials, that is, which also comprise a diagnostic unit, shall be equally covered by the generic term diagnostic apparatus.

A problem with an ECG apparatus is that, despite the internationally standardized color coding and terms used for the electrodes and/or the electrode cables, the electrodes are confused (compare A. Rudiger, L. Schöb: ECG of a young patient with influenzal complaints, who is suspected of having ischaemia, Switzerland Med Forum No. 28, 11.7.2001, Page 741 et seq.). At least in simple cases, current signal evaluation methods are able to detect in the potential curves over time if electrodes were confused (Kors J. A., van Herpen G.: Accurate automatic detection of electrode interchange in the electrocardiogram, Am J. Cardiol, 15.8.2001; 88(4):396-9).

If the potential curves are abnormally changed or noisy, these methods quickly reach their limits. Especially when chest leads (V1-V6) are made, the physical distance between the electrodes is so small that a safe detection of confused electrodes is very difficult.

US 5,042,498 as well as the other patent family members EP 0 450 350 A1, DE 69119133 T2 and JP 4227229 A disclose an intelligent ECG system comprising the features of the preamble of claim 1. The electrodes used comprise a pad with a post and a snap connector which clamps the post and includes an LED anchored in the top central portion of the snap connector. A lead with three wires connects the snap connector to the ECG apparatus. One wire contacts the snap connector itself. The other two wires contact the LED. A detector circuit in the ECG apparatus supplies via a wire in the lead a constant current to the post. If the voltage drop between the post and the body of a patient is too great, a poor contact is assumed and the LED is switched on. The wire that connects the LED to a ground connection in the ECG apparatus may be used as a shielding for the wire providing the ECG signal.The LED can also be driven by a lead sequencer, wherein the LEDs are illuminated sequentially to guide a technician through the task of placing a group of electrodes in their proper locations on the patient. DE 100 29 205 A1 discloses an apparatus for measuring physiological parameters. ECG measuring electrodes are movably positioned in a belt system. The belt system further comprises an electronic measurement system, a device for the wireless transmission of the digitalized measured signals, a power supply unit and an antenna. The electrodes are arranged movably and include an LED display. The receiving station detects by means of a program whether the individual electrodes are possibly not connected or wrongly positioned, which is signalized, for example, by a red LED display on the respective electrode.

It is the object of the invention to provide a user-friendly ECG system and a corresponding method. This method is achieved with the teaching of the independent claims.

Preferred embodiments of the invention are defined in the dependent claims.

A running light on the ECG electrodes has the advantage that the running light is easy to implement in an ECG apparatus. The "evaluation" is more or less made by the user, so that no complicated algorithms are necessary. The type and quality of the recorded ECG signals has no influence on the accuracy of the evaluation.

The evaluation may also be made by an image recorder (e.g. simple digital camera or web cam) and an evaluation software.

A preferred embodiment of the invention will be explained in more detail below, with reference to the accompanying drawing. In the drawing:

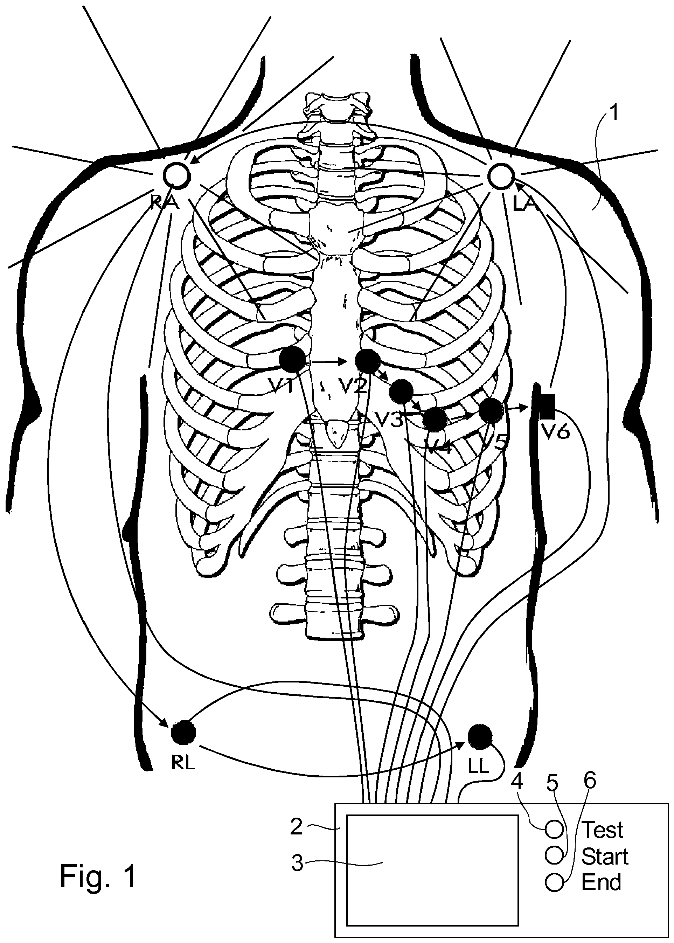

Fig. 1 shows a typical arrangement of electrodes for recording an ECG by means of an ECG apparatus according to the invention.

Fig. 1 shows the typical arrangement of electrodes on the upper part of the body of a patient 1, which are connected to an ECG apparatus 2. To provide a better overview, not only the contours of the upper part of the body are drawn in, but also a part of the spine, the thorax and the sternum. Ten electrodes are used for picking off the bioelectric potentials, namely, V1, V2, V3, V4, V5, V6, LA, RA, RL and LL according to the US standard nomenclature. Each electrode is provided with a light source, specifically an LED. Electrodes whose light source is switched off are represented by black circles, or a black quadrangle in the case of electrode V6. The electrodes whose light source is switched on, i.e. LA and RA, are represented by black rings. In this document the ECG apparatus 2 together with the ten electrodes will be designated as an ECG system. The ECG apparatus 2 may comprise a display means 3, which may be a screen, a printer and/or a plotter.

This invention helps a user to check proper placement of the electrodes quickly and comfortably after having fixed the electrodes on the patient 1. The reader will appreciate that it is sometimes necessary to measure ECGs in error-prone situations, e. g. in accidents at nighttime, when the user is tired. It is important that the user notices a possible confusion of the electrodes as quickly as possible after placement. To this end a so-called running light mode is provided in which the electrodes are switched on and off again like a running light. This means that the light sources assigned to the electrodes are switched on in a predetermined sequence and are switched off again in this sequence, whereby a different number of light sources can light up simultaneously.

If a succeeding light source is switched on as the light source directly preceding the succeeding light source is switched off, and the last light source is followed by the first light source, always one single light source is switched on.

If the xth light source in the sequence is switched on as the (x-2)th light source is switched off, two light sources light up simultaneously. This embodiment is illustrated in Fig. 1, in which the light sources assigned to the two electrodes LA and RA light up.

In another embodiment the overlap between two light sources may be smaller, e. g. 5% to 40 %, in particular 20 % of the time between switching on the x-th and the (x+1)th light source. This may be referred to as a slight overlap. The x-th and the (x+1)th light source may be designated neighboring light sources.

In the extreme case all light sources may be switched on before the first light source is switched off again, so that there is a time when all light sources light up. Also, the light sources may be switched on successively and switched off simultaneously, or switched on simultaneously and switched off successively.

The running light mode may be described as a sequence of states, wherein no, one, several or all light sources may be switched on in each state. Each switching on and off of a light source is then a state change.

It should be expedient to switch the light sources on at an interval of approximately 0.5 to 2 seconds, for example one second and to chose this time interval also for switching them off. Thus, with ten electrodes and light sources and without an interval between the last and the first light source, a period of 5 to 20 seconds, for example 10 seconds is obtained, corresponding to a frequency of 0.2 to 0.05 Hz, for example 0.1 Hz. Especially in embodiments in which many or all light sources light up simultaneously, there should be a pause between the last and the first light source, that is, before the sequence of switching the light sources on and off is repeated.

The keys may allow a user to adjust the interval between switching on neighboring light sources for example from 0.3 to 3 seconds. Also the overlap may be adjusted by the keys from 0% to 900% of the interval between switching on neighboring light sources. 0% overlap means that the (x+1)-th light source is switched on when the x-th light source is switched off. Skilled persons will appreciate that the ECG apparatus is micro processor controlled and that the micro processor is a suitable means for controlling the switching on and off of the light sources. The micro processor or a memory within the ECG apparatus may store different settings for the interval and the overlap.

As can be seen in Fig. 1, the electrodes approximately form a Latin letter "e". The sequence starts on the left at the transverse beam of the e and ends on the righthand bottom at the end of the bow of the e. Thus, the sequence is: V1, V2, V3, V4, V5, V6, LA, RA, RL, LL, which is shown by arrows in Fig. 1.

If the ECG apparatus 2 is switched on, for example, by means of the starter button 5, in an embodiment not forming part of the present invention the ECG apparatus 2 can automatically switch into the running light mode. Subsequently, a user can secure the ten electrodes or a part thereof on the patient 1, while the running light helps to avoid a confusion of the electrodes. Next, the user can exit the running light mode by pressing the stop button 6. By means of the test key 4 the running light mode may be activated again.According to the invention, the ECG apparatus 2 automatically switches to the running light mode, after the electrodes have been secured on the patient 1 and successful electrode contact quality check was performed by the ECG apparatus 2. After the ECG apparatus 2 has been switched on, it starts to check electrode contact quality, up until all or a predefined subset of electrodes is secured with sufficient contact quality to the patient 1. During this period the LEDs may indicate poor-contact electrodes. After switching to the running light mode, the user can quickly and reliably check the proper sequence of the electrodes.

There are many ways known in the art to perform an electrode contact quality check, cf e. g. US 5,042,498. In further embodiments not forming part of the present invention, the user may select as to whether the ECG apparatus 2 switches directly into the running light mode or does the electrode contact quality check first after it has been switched on. The user may also cancel a unsuccessful contact quality check and switch to the running light mode.

### List of Reference Numbers

- 1: patient
- 2: ECG apparatus
- 3: display means
- 4, 5, 6: keys
- V1, V2, V3, V4, V5, V6, LA, RA, RL, LL: electrodes

## Claims

1. An ECG system, comprising:
an ECG apparatus (2); and
a plurality of electrodes (V1-V6, LA, RA, RL, LL) each being provided with a light source, wherein both the electrodes and the light sources are electrically connected to the ECG apparatus (2), the ECG apparatus (2) being adapted to perform an electrode contact quality check, and the ECG apparatus (2) being adapted to switch on and off the light sources in a predetermined sequence in a running light mode;
**characterized in that**
the ECG apparatus (2) is adapted to automatically switch into the running light mode after the electrodes have been secured on the patient (1) and a successful electrode contact quality check was performed by the ECG apparatus (2).

2. The ECG system according to claim 1, **characterized in that** the defined sequence consists in passing through an e-shaped arrangement of the electrodes from the left end of the transverse beam of the e to the right lower end of the bow of the e, that is, in a sequence V1, V2, V3, V4, V5, V6, LA, RA, RL, LL.

3. The ECG system according to one of the preceding claims, **characterized in that** one period of the running light mode lasts approximately 10 seconds.

4. The ECG system according to one of the preceding claims, **characterized in that** a state change in the defined sequence takes place after approximately one second.

5. A method of operating an ECG system, which comprises an ECG apparatus (2) and a plurality of electrodes (V1-V6, LA, RA, RL, LL) each being provided with a light source, wherein both the electrodes and the light sources are electrically connected to the ECG apparatus (2), and wherein the ECG apparatus is micro processor controlled, the method comprising:
switching the light sources on and off in a predetermined sequence under control of the micro processor so as to give the impression of a running light;
wherein the ECG apparatus (2) automatically switches into the running light mode after the electrodes have been secured on the patient (1) and a successful electrode contact quality check was performed by the ECG apparatus (2).

6. The method according to claim 5, **characterized in that** the defined sequence consists in passing through an e-shaped arrangement of the electrodes from the left end of the transverse beam of the e to the right lower end of the bow of the e, that is, in a sequence V1, V2, V3, V4, V5, V6, LA, RA, RL, LL.

7. The method according to one of claims 5 to 6, **characterized in that** one period of the running light mode lasts approximately 10 seconds.

8. The method according to one of claims 5 to 7, **characterized in that** a state change in the defined sequence takes place after approximately one second.

## Patentansprüche

1. Ein EKG-System mit:
einem EKG-Gerät (2); und
einer Vielzahl von Elektroden (V1-V6, LA, RA, RL, LL), wobei jede mit je einer Lichtquelle ausgestattet ist, wobei sowohl die Elektroden als auch die Lichtquellen mit dem EKG-Gerät (2) elektrisch verbunden sind, wobei das EKG-Gerät (2) angepasst ist, um eine Elektrodenkontakt-Qualtitätsprüfung durchzuführen und das EKG-Gerät (2) angepasst ist, um die Lichtquellen in einer festgelegten Folge in einem Lauflichtmodus an- und auszuschalten;
**dadurch gekennzeichnet, dass**
das EKG-Gerät (2) angepasst ist, um automatisch in den Lauflichtmodus zu schalten, nachdem die Elektroden auf dem Patienten (1) befestigt worden sind und eine erfolgreiche Elektrodenkontakt-Qualtitätsprüfung vom EKG-Gerät (2) durchgeführt worden ist.

2. Das EKG-System gemäß Anspruch 1, **dadurch gekennzeichnet, dass** die festgelegte Folge darin besteht, die e-förmige Anordnung der Elektroden vom linken Ende des Querbalkens des e zum rechten Ende des Bogens des e, also in der Folge V1, V2, V3, V4, V5, V6, LA, RA RL, LL zu durchlaufen.

3. Das EKG-System gemäß einem der vorangehenden Ansprüche, **dadurch gekennzeichnet, dass** eine Periode des Lauflichtmodus etwa 10 Sekunden dauert.

4. Das EKG-System gemäß einem der vorangehenden Ansprüche, **dadurch gekennzeichnet, dass** ein Zustandswechsel in der festgelegten Folge nach etwa einer Sekunde erfolgt.

5. Ein Verfahren zum Betreiben eines EKG-Systems, das ein EKG-Gerät (2) und eine Vielzahl von Elektroden (V1-V6, LA, RA, RL, LL) umfasst, die mit je einer Lichtquelle ausgestattet sind, wobei sowohl die Elektroden als auch die Lichtquellen mit dem EKG-Gerät (2) elektrisch verbunden sind und wobei das EKG-Gerät durch einen Mikroprozessor gesteuert wird, wobei das Verfahren folgendes umfasst:
Ein- und Ausschalten der Lichtquellen in einer vorher festgelegten Folge unter Steuerung des Mikroprozessors, so dass der Eindruck eines Lauflichts entsteht;
wobei
das EKG-Gerät (2) automatisch in den Lauflichtmodus schaltet, nachdem die Elektroden auf dem Patienten (1) befestigt worden sind und eine erfolgreiche Elektrodenkontakt-Qualtitätsprüfung vom EKG-Gerät (2) durchgeführt worden ist.

6. Das Verfahren gemäß Ansprüch 5, **dadurch gekennzeichnet, dass** die festgelegte Folge darin besteht, die e-förmige Anordnung der Elektroden vom linken Ende des Querbalkens des e zum rechten Ende des Bogens des e, also in der Folge V1, V2, V3, V4, V5, V6, LA, RA RL, LL zu durchlaufen.

7. Das Verfahren gemäß einem der Ansprüche 5 bis 6, **dadurch gekennzeichnet, dass** eine Periode des Lauflichtmodus etwa 10 Sekunden dauert.

8. Das Verfahren gemäß einem der Ansprüche 5 bis 7, **dadurch gekennzeichnet, dass** ein Zustandswechsel in der festgelegten Folge nach etwa einer Sekunde erfolgt.

## Revendications

1. Système ECG, comprenant :
un appareil ECG (2); et
une pluralité d'électrodes (V1-V6, LA, RA, RL, LL) donc chacune est pourvue d'une source lumineuse, dans lequel tant les électrodes que les sources lumineuses sont reliées électriquement à l'appareil ECG (2), l'appareil ECG (2) étant conçu pour effectuer un contrôle de qualité de contact d'électrode et l'appareil ECG (2) étant conçu pour allumer et éteindre les sources lumineuses selon une séquence prédéterminée dans un mode de feu de circulation;
**caractérisé en ce que**:
l'appareil ECG (2) est conçu pour basculer automatiquement dans le mode de feu de circulation une fois les électrodes fixées sur le patient (1) et un contrôle de qualité de contact d'électrode réussi effectué par l'appareil ECG (2).

2. Système ECG selon la revendication 1, **caractérisé en ce que** la séquence définie consiste à traverser un agencement en forme de e des électrodes depuis l'extrémité gauche de la barre transversale du e jusqu'à l'extrémité inférieure droite de la courbe du e, c'est à dire selon une séquence V1, V2, V3, V4, V5, V6, LA, RA, RL, LL.

3. Système ECG selon l'une quelconque des revendications précédentes, **caractérisé en ce qu'**une période du mode de feu de circulation dure approximativement 10 secondes.

4. Système ECG selon l'une quelconque des revendications précédentes, **caractérisé en ce qu'**un changement d'état dans la séquence définie se produit après approximativement une seconde.

5. Procédé d'utilisation d'un système ECG qui comprend un appareil ECG (2) et une pluralité d'électrodes (V1-V6, LA, RA, RL, LL) dont chacune est pourvue d'une source lumineuse, dans lequel tant les électrodes que les sources lumineuses sont reliées électriquement à l'appareil ECG (2) et dans lequel l'appareil ECG est commandé par microprocesseur, le procédé comprenant:
l'allumage et l'extinction des sources lumineuses selon une séquence prédéterminée sous contrôle du microprocesseur de façon à donner l'impression d'un feu de circulation
dans lequel l'appareil ECG (2) bascule automatiquement dans le mode de feu de circulation une fois les électrodes fixées sur le patient (1) et un contrôle de qualité de contact d'électrode réussi effectué par l'appareil ECG (2).

6. Procédé selon la revendication 5, **caractérisé en ce que** la séquence définie consiste à traverser un agencement d'électrodes en forme de e de l'extrémité gauche de la barre transversale du e jusqu'à l'extrémité inférieure droite de la courbe du e, c'est-à-dire selon une séquence V1, V2, V3, V4, V5, V6, LA, RA, RL, LL.

7. Procédé selon l'une quelconque des revendications 5 à 6, **caractérisé en ce qu'**une période du mode de feu de circulation dure approximativement 10 secondes.

8. Procédé selon l'une quelconque des revendications 5 à 7, **caractérisé en ce qu'**un changement d'état dans la séquence définie se produit après approximativement une seconde.
